# EUROPEAN PATENT APPLICATION

(11) **EP 4 685 223 A1**
(43) Date of publication of application: **28.01.2026**
(21) Application number: 23928570.3
(22) Date of filing: 20.03.2023
(51) Int. Cl.: C12M 1/34, C12Q 1/02

(54) **INSPECTION DEVICE AND INSPECTION METHOD**

(71) Applicant: HITACHI HIGH-TECH CORPORATION, Tokyo 105-6409 (JP)
(72) Inventor: SUGIYAMA, Kiyotaka, Tokyo 105-6409 (JP)
(74) Representative: MERH-IP Matias Erny Reichl Hoffmann Patentanwälte PartG mbB
(86) International application number: PCT/JP2023/010897
(87) International publication number: WO 2024/194987

(57) **Abstract**

The present invention comprises: an imaging unit that captures an image of the inside of a container that retains a liquid containing fine particles; and an image-processing unit. The image-processing unit calculates a first statistic related to the spread of luminance values in the image, excludes a region having abnormal luminance values in the image on the basis of the result of comparison of the luminance values in the image with a predetermined luminance threshold, performs a correction process of sharpening the image from which the region having abnormal luminance values has been excluded, calculates a second statistic related to the spread of luminance values in the image subjected to the correction process, determines the validity of the correction process on the basis of the result of comparison of the ratio of the first statistic and the second statistic with a predetermined statistical ratio threshold, determines the degree of proliferation of fine particles on the basis of a feature amount of the image for which the correction process has been determined to be valid, and outputs the result of determination of the degree of proliferation. As a result, it is possible to more accurately extract bacteria and detect a region in which bacteria corresponding to the degree of proliferation are present regardless of the difference in the degree of proliferation or the presence or absence of foreign matters other than the bacteria included in an image including a particulate object to be measured such as growing bacteria.

## Description

### Technical Field

The present invention relates to an inspection device and an inspection method.

### Background Art

International epidemics of infectious diseases are a major concern for human health worldwide. In recent years, various emerging infectious diseases have been spreading one after another, and in response, new inspection methods for specifying and detecting causative viruses and causative bacteria, and new vaccines have developed. For example, in bacterial infectious diseases, currently, the emergence of multidrug-resistant bacteria has become a serious problem on a global scale, and the US CDC states that carbapenem-resistant Acinetobacter, carbapenem-resistant Enterobacteriaceae, Clostridiodis difficile, and the fungus Candida auris are major threats. When such cluster infection of resistant bacteria occurs, since the risk of death of the patient is high, it is important to quickly perform identification and sensitivity measurement of the causative bacteria, detect drug-resistant bacteria, and quickly perform appropriate treatment. As an identification test (ID) for identifying the causative bacteria, a method using mass spectrometry is mainly used, and it is now possible to perform tests within one hour from colony or positive blood culture samples.

As a susceptibility testing (AST) for evaluating sensitivity of bacteria to drugs, a test using turbidity measurement is mainly used, but to speed up the test, a method for capturing images of bacteria with a microscope and detecting a degree of proliferation of the bacteria with a sensitivity higher than that in a method in the related art has been put into practical use. In general, the bacteria proliferate twice every time according to a certain proliferation period. Therefore, since the number of bacteria present in the image captured by the microscope also proliferates twice every time and exponentially increases, a wide dynamic range is required to accurately capture the temporal change of the proliferation.

As a technique in the related art according to detection of the degree of proliferation of bacteria from an image, for example, a technique described in PTLs 1 to 3 is known.

PTL 1 discloses a method for acquiring an image of bacteria, extracting a bacterium region by an image process, and detecting a region where bacteria are present. Specifically, the bacterium region is detected by synthesizing an image, which is obtained by binarizing a bacterial image with a certain threshold, and an image obtained by performing a contour extraction process and then binarizing the image. In this method, the number of bacteria in the image is small, and when the individual bacteria appear in isolation in the image, it is possible to accurately extract the bacterium region.

PTL 2 discloses a method for adjusting a contrast by histogram extension of an image luminance value such that brightness of an output image does not fluctuate even if brightness of an input image changes.

PTL 3 discloses a method for detecting a foreign matter by an image. Specifically, there is disclosed a method for adjusting a contrast by histogram extension of an image luminance value in order to implement good foreign matter detection. In this method, when there is a pixel defect, contrast enhancement can be performed by correcting an influence of a defective pixel.

### Citation List

### Patent Literature

PTL 1: US Patent No. 10902592
PTL 2: JP2001-119683A
PTL 3: JP2006-194869A

### Summary of Invention

### Technical Problem

However, the technique in the related art has the following problems.

In the related art described in PTL 1, when the proliferation of the bacteria progresses and the bacteria do not appear in isolation, it is difficult to separate and correctly detect the bacteria one by one in the image. When the proliferation of the bacteria progresses and the bacteria do not appear in isolation, it is difficult to separate a boundary of the adjacent bacteria because contours of the bacteria are common, and a plurality of bacteria are recognized as one large mass. Therefore, it is not easy to isolate, detect, and recognize individual bacteria and detect the number of bacteria in a sufficiently wide dynamic range. In addition, since an amount of light irradiated to a detection unit is reduced due to the proliferated bacteria, and a difference in shading between image luminance values of a background region and the bacterium region in the image is reduced, it is necessary to perform a process of appropriately adjusting the contrast and extracting the bacteria, but it is difficult to correctly detect the bacterium region in a case where the number of bacteria in the image is different and in a case where a foreign matter is present in the image.

In addition, although the technique in the related art described in PTL 2 is intended to accurately detect a foreign matter other than a target object, it is not intended to accurately detect a target object, and is not suitable for object detection in an image in which the number of measurement target objects such as bacteria included in the image changes in a range of several digits.

In addition, in the related art described in PTL 3, when a foreign matter other than a target object appears in the image, contrast enhancement is performed by correction, and it is possible to more accurately execute an image process by performing an enhancement process while excluding the influence of the foreign matter, but it is unclear whether the degree of the corrected contrast enhancement is appropriate, and there is room for improvement.

The invention has been made in view of the above, and an object of the invention is to provide an inspection device and an inspection method capable of more accurately extracting bacteria and detecting a region in which bacteria corresponding to the degree of proliferation are present regardless of the difference in the degree of proliferation or the presence or absence of foreign matters other than the bacteria included in an image including a particulate object to be measured such as proliferating bacteria.

### Solution to Problem

The present application includes a plurality of units for solving the above problems, and examples thereof include: an imaging unit configured to capture an image of an inside of a container that retains a liquid containing fine particles; and an image-processing unit configured to perform an image process on the image captured by the imaging unit. The image-processing unit calculates a first statistic related to a spread of a luminance value of the image, excludes a region having an abnormal luminance value in the image based on a result of comparison of the luminance value of the image with a predetermined luminance threshold, performs a correction process of sharpening the image from which the region having the abnormal luminance value is excluded, calculates a second statistic related to the spread of the luminance value of the image subjected to the correction process, determines validity of the correction process based on a result of comparison of a ratio of the first statistic and the second statistic with a predetermined statistical ratio threshold, determines a degree of proliferation of the fine particles based on a feature value of the image for which the correction process is determined to be valid, and outputs a result of determination of the degree of proliferation.

### Advantageous Effects of Invention

According to the invention, it is possible to more accurately extract bacteria and detect a region in which bacteria corresponding to the degree of proliferation are present regardless of the difference in the degree of proliferation or the presence or absence of foreign matters other than the bacteria included in an image including a particulate object to be measured such as proliferating bacteria.

### Brief Description of Drawings

[FIG. 1] FIG. 1 is a diagram schematically illustrating a configuration of a bacterium inspection device as an example of an inspection device.
[FIG. 2] FIG. 2 is a functional block diagram schematically illustrating a configuration of a processing function of an image-processing unit.
[FIG. 3] FIG. 3 is a diagram illustrating a feature of an image according to a degree of proliferation of bacteria, and is a diagram illustrating an image before culture (before proliferation of the bacteria).
[FIG. 4] FIG. 4 is a diagram illustrating a feature of an image according to the degree of proliferation of the bacteria, and is a diagram illustrating an image when the proliferation of the bacteria has progressed to some extent.
[FIG. 5] FIG. 5 is a diagram illustrating a feature of an image according to the degree of proliferation of the bacteria, and is a diagram illustrating an image when the proliferation of the bacteria has significantly progressed.
[FIG. 6] FIG. 6 is a diagram illustrating an example of a histogram of a luminance value of an image.
[FIG. 7] FIG. 7 is a diagram illustrating an example of an image of the bacteria when a correction process (contrast enhancement) at an appropriate intensity is performed on the image of the bacteria illustrated in FIG. 4.
[FIG. 8] FIG. 8 is a diagram illustrating a histogram corresponding to the images of the bacteria in FIGS. 4 and 7 when the histogram is extended as the correction process (contrast enhancement).
[FIG. 9] FIG. 9 is a diagram illustrating a histogram of luminance before and after a histogram enhancement process when there is an object appearing dark in an image.
[FIG. 10] FIG. 10 is a diagram illustrating a histogram of luminance before and after the histogram enhancement process when almost no bacteria appear in an image.
[FIG. 11] FIG. 11 is a diagram illustrating an example of an image after a contrast enhancement process (correction process) as illustrated in FIG. 10.
[FIG. 12] FIG. 12 is a flowchart illustrating a content of a bacterium detection process in an image-processing unit of a bacterium inspection device according to a first embodiment.
[FIG. 13] FIG. 13 is a diagram schematically illustrating a single bacterium in an image.
[FIG. 14] FIG. 14 is a diagram illustrating a relationship between a position of a pixel in FIG. 13 and a luminance value.
[FIG. 15] FIG. 15 is a diagram illustrating an effect of an image process in the image-processing unit.
[FIG. 16] FIG. 16 is a diagram illustrating an example of an image process result output to an input and output unit of a control device.
[FIG. 17] FIG. 17 is a diagram illustrating an example of a histogram of a luminance when many foreign matters appear in an image.
[FIG. 18] FIG. 18 is a flowchart illustrating a part of a content of an image process according to a second embodiment.

### Description of Embodiments

The embodiment discloses, for example, a technique of performing contrast enhancement on an image of fine particles captured by a microscope in a well holding a liquid containing the fine particles, determining validity of the contrast enhancement, and accurately determining the degree of proliferation of the fine particles. Hereinafter, embodiments according to the disclosure will be described with reference to the drawings. Although the accompanying drawings illustrate specific embodiments based on principles of the present technique, the embodiments are provided for easily understanding the present technique and are not to be used to limit the interpretation of the present disclosure. In the embodiments and the drawings, components having the same functions are denoted by the same reference numerals, and repeated description may be omitted. Examples of the fine particle include bacteria, archaea, fungi, and biological cells, and bacteria will be described here as one type of the fine particle.

### <First Embodiment>

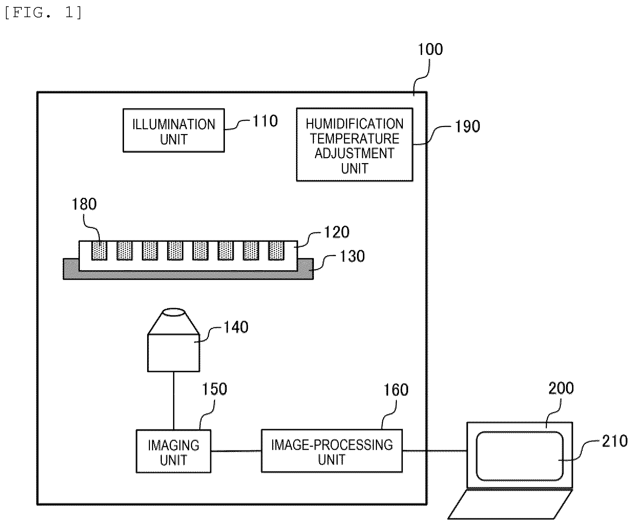

A first embodiment of the invention will be described in detail with reference to FIGS. 1 to 16.

### (Bacterium Inspection Device 100)

FIG. 1 is a diagram schematically illustrating a configuration of a bacterium inspection device as an example of an inspection device according to the embodiment.

In FIG. 1, a bacterium inspection device 100 schematically includes an illumination unit 110, an inspection plate 120, a stage 130, an objective lens 140, an imaging unit 150, an image-processing unit 160, and a humidification temperature adjustment unit 190. The bacterium inspection device 100 further includes a control device 200 that controls the overall operation of the bacterium inspection device 100.

The inspection plate 120 has one or more wells, preferably 96 wells or 384 wells, and a sample solution 180 is held in each well. The sample solution 180 contains a bacterial sample, and may also contain a medium for bacterial proliferation or one or more antibacterial agents having a pre-adjusted concentration. Since the inspection plate 120 changes depending on the type of bacteria as an inspection target, a type of drug, or the like, it is preferable that a plurality of the inspection plates 120 different for each inspection are introduced into the bacterium inspection device 100. The inspection plate 120 is preferably distinguished by a patient ID, bacteria as the inspection target, a type of drug to be used, or the like, and is individually identified by, for example, a barcode label, a two-dimensional code, and

### RFID.

The illumination unit 110 emits light toward the inspection plate 120. The illumination unit 110 may use a light source such as a white light source such as a lamp, or an LED that includes light in a specific wavelength region. The light emitted from the illumination unit 110 to the inspection plate 120 passes through each well in the inspection plate 120 and the sample solution 180, is condensed by the objective lens 140, and is imaged by the imaging unit 150. For example, by moving the stage 130 under the control of the control device 200 and changing relative positions of the wells of the inspection plate 120 and the imaging unit 150, different wells can be imaged. In addition, the control device 200 also controls an imaging operation, which is executed at preset time intervals (for example, every 30 minutes). An obtained image is processed by the image-processing unit 160 and transmitted to the control device 200.

A focal point of the objective lens 140 is preferably aligned with a well bottom surface of the inspection plate 120, and may be aligned with an inside of the sample solution 180 separated from the bottom surface. Images may be captured at a plurality of locations in the well, or a plurality of images of the inside of the sample solution 180 separated from the well bottom surface of the inspection plate 120 may be captured.

The humidification temperature adjustment unit 190 adjusts a temperature and a humidity of the entire imaging system for acquiring an image, and for example, by adjusting the temperature to about 35 °C and the humidity to about 30%, the bacterial proliferation in the inspection plate 120 is efficiently advanced.

The control device 200 is, for example, a general computer, controls the entire bacterium inspection device 100 including the imaging unit 150 and the image-processing unit 160, and includes an input and output unit 210 that has a function of inputting various types of information such as measurement conditions and setting values related to the inspection to the bacterium inspection device 100, and receiving and outputting (displaying) inspection results. The input and output unit 210 is, for example, a display, a mouse, a keyboard, or a touch panel.

### (Image-Processing Unit 160)

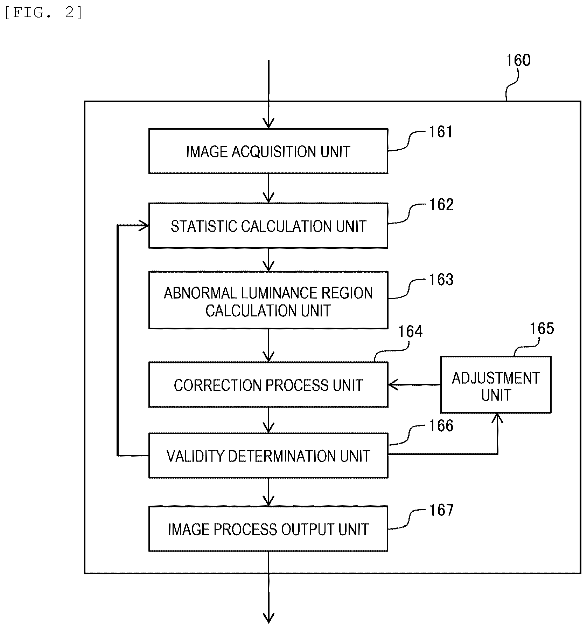

FIG. 2 is a functional block diagram schematically illustrating a configuration of a processing function of the image-processing unit.

In FIG. 2, the image-processing unit 160 performs a bacterium detection process of calculating and outputting a degree of proliferation of bacteria based on an image obtained by the imaging unit 150 under the control of the control device 200, and includes an image acquisition unit 161 that acquires an image captured by the imaging unit 150, a statistic calculation unit 162 that calculates a statistic related to a spread of a luminance value of the image or the like acquired by the image acquisition unit 161, an abnormal luminance region calculation unit 163 that excludes a region having an abnormal luminance value of the image based on a result of comparison of the luminance value of the image with a predetermined luminance threshold, a correction process unit 164 that performs a correction process of sharpening the image excluding the region having the abnormal luminance value, a validity determination unit 166 that determines validity of the correction process of the correction process unit 164 based on a result of comparison of a ratio of a statistic of the image before the correction process to a statistic of the image after the correction process with a predetermined statistical ratio threshold, and an adjustment unit 165 that adjusts a reference value used for the correction process of the correction process unit 164 when the validity determination unit 166 determines that the correction process of the correction process unit 164 is not valid, and an image process output unit 167 that determines the degree of proliferation of the fine particles based on a feature value of the image for which the correction process of the correction process unit 164 is determined to be valid by the validity determination unit 166, and outputs the result of determination.

Hereinafter, the details of a bacterium inspection process by the bacterium inspection device 100 (control device 200) including the image-processing unit 160 (bacterium detection process) will be described.

### (Basic Principle)

First, a basic principle of the bacterium inspection process by the bacterium inspection device according to the embodiment will be described.

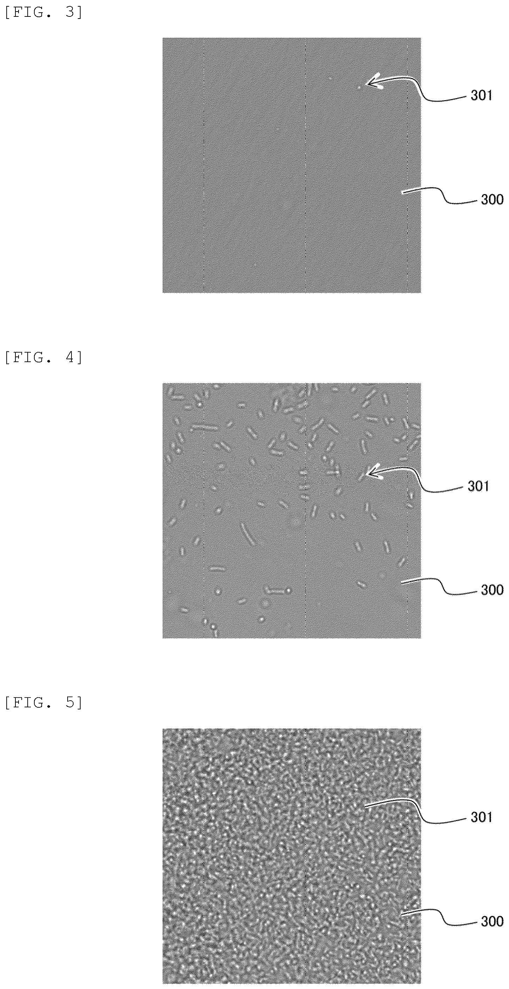

FIGS. 3 to 5 are diagrams illustrating features of the images according to the degree of proliferation of bacteria. FIG. 3 illustrates an image before culture (before proliferation of bacteria). FIG. 4 illustrates an image when the proliferation of bacteria has progressed to some extent. FIG. 5 illustrates an image when the proliferation of bacteria has greatly progressed. In FIGS. 3 to 5, a grayscale image of 256 gradations with 8-bit is illustrated.

As illustrated in FIGS. 3 to 5, the image includes a region of a background 300, a region of bacteria 301, and a region of a foreign matter other than the background 300 and the bacteria 301 as main components.

For example, as illustrated in FIG. 3, in the image before culture (before proliferation of bacteria), most of the image is formed by the background 300, and the bacteria 301 are minute regions.

As shown in FIG. 4, in the image after culture (when the proliferation of the bacteria progresses to some extent), the bacteria are divided and proliferate, and the region of the bacteria 301 is increased. Even in this case, the region of the background 300 can be checked.

Further, as shown in FIG. 5, when the proliferation progresses greatly, the region of the background 300 is hardly checked, and most of the image is filled with the region of the bacteria 301.

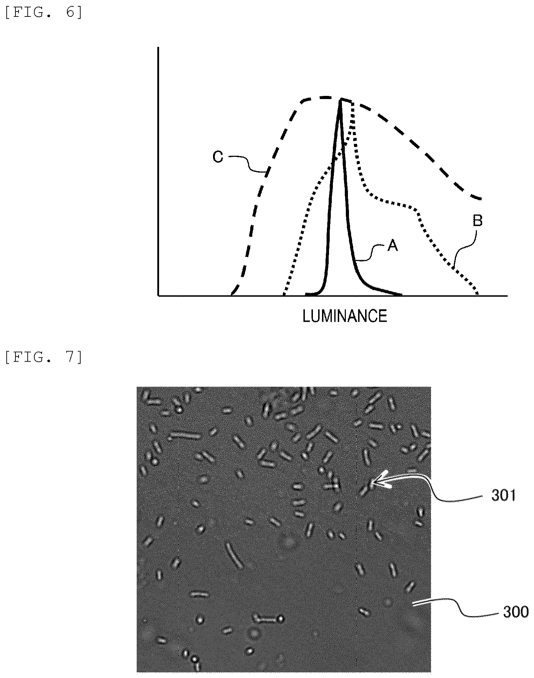

FIG. 6 is a diagram illustrating an example of a histogram of a luminance value of an image, in which a horizontal axis indicates a luminance in a range of 0 to 255 and a vertical axis indicates the number of pixels of a corresponding luminance value. FIG. 6 illustrates a histogram A corresponding to the image before the proliferation of bacteria (FIG. 3), a histogram B corresponding to the image when the proliferation of bacteria has progressed to some extent (FIG. 4), and a histogram C corresponding to the image when the proliferation of bacteria has greatly progressed (FIG. 5).

The histogram A corresponding to the image before the proliferation of bacteria illustrates a steep shape with a small difference between the minimum value and the maximum value. This is because most of the image is the region of the background 300. When the bacteria start to proliferate, the region of the bacteria 301 increases. Since the inside of the region of the bacteria 301 is brighter than the background and a contour of the bacteria 301 is darker than the background, a width of the histogram increases as illustrated in the histogram B when the bacteria proliferate. When the bacteria further proliferate, a region where an amount of light locally increases or decreases occurs due to scattered light, and thus the width is further increased as in the histogram C. As the bacteria proliferate, the histogram of the image changes like the histograms A, B, and C, and not only the width of the histogram but also the peak position changes. On the other hand, in addition to the degree of proliferation of the bacteria, the change in the histogram is complicated depending on the bacterial species or bacterial strain of the bacteria, and thus the histogram does not have a regular pattern. In addition, for example, when foreign matters other than bacteria, such as dust, air bubbles, and biological cells other than bacteria are present in the image, the change in the histogram becomes more complicated. As described above, in the related art, it is difficult to execute a correction process such as a contrast enhancement process at an appropriate intensity on various images in which the histogram changes in a complicated manner depending on various conditions.

### (Example of Correction Process at Appropriate Intensity)

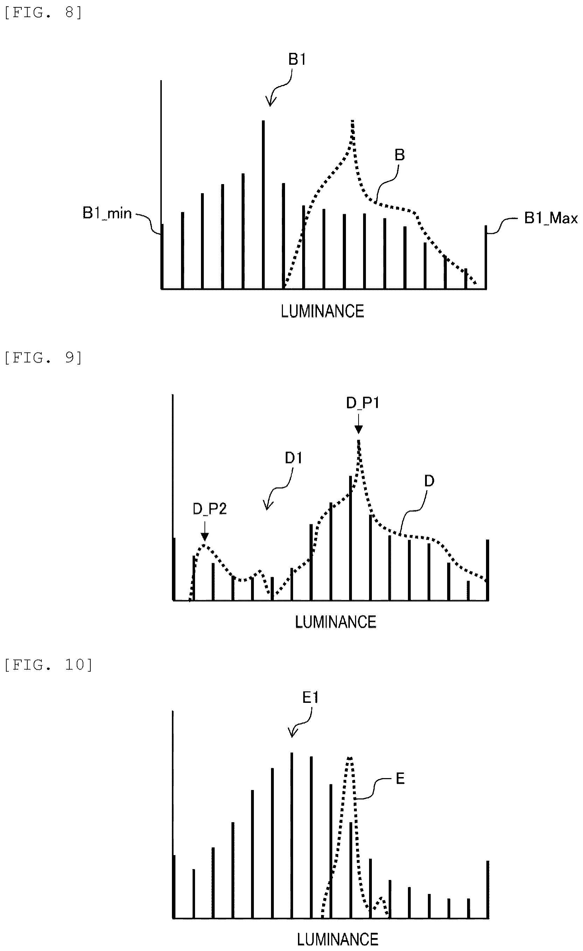

FIG. 7 is a diagram illustrating an example of an image of the bacteria when a correction process (contrast enhancement) at an appropriate intensity is performed on the image of the bacteria illustrated in FIG. 4. FIG. 8 is a diagram illustrating a histogram corresponding to the images of the bacteria in FIGS. 4 and 7 when the histogram is extended as the correction process (contrast enhancement).

As illustrated in FIG. 7, when the contrast enhancement is executed as the correction process on the image of the bacteria illustrated in FIG. 4 and the contrast enhancement process is performed as expected, a difference in shading between a region having a high luminance inside the bacteria 301 and a region having a low luminance of the contour of the bacteria 301 is enhanced as compared with FIG. 4. That is, since a luminance difference between the region of the background 300 and the region of the bacteria 301 in the image of the bacteria illustrated in FIG. 7 is sufficiently large, it is easier to calculate the feature value of the image of the bacteria by distinguishing the two. As one of correction process methods, for example, extension of a histogram disclosed in JP2001-119683A (PTL 2) can be used.

As shown in FIG. 8, when the histogram is extended as the contrast enhancement on the image of the bacteria shown in FIG. 4, first, the number of pixels are accumulated in descending order of the luminance value for the histogram B of the luminance before correction, and the luminance value in which an accumulated value exceeds a reference is set as a lower limit value B1_min of a saturation luminance. As the reference value, it is possible to select a ratio of a target image to all pixels. For example, when a ratio of 1% with respect to an image of 100 × 100 pixels is set as the reference value, the reference value is 100, and a minimum value of the luminance value at which the accumulated value of the number of pixels exceeds 100 is the lower limit value B1_min of the saturation luminance. That is, an intensity of the correction process (contrast enhancement process) is changed by increasing or decreasing the ratio (reference value). Similarly, secondly, the number of pixels is accumulated in ascending order of the luminance value for the histogram B of the luminance before correction, and the luminance value in which the accumulated value exceeds the reference is set as an upper limit value B1_Max of the saturation luminance. Finally, the luminance of pixels corresponding to the lower limit value B1_min of the saturation luminance and the upper limit value B1_Max of the saturation luminance are regarded as the minimum and maximum of the image luminance (for example, 0 and 255 in the case of the image of 8-bit and 256 gradation), and the histogram is uniformly extended to obtain a histogram B1 after correction. By extending the histogram B before correction, the histogram B1 after correction becomes more discrete.

### (Example of Correction Process at Inappropriate Intensity)

As shown in FIG. 7 and FIG. 8, there are cases where the correction process at the appropriate intensity is performed, but there are cases where the enhancement process as expected is not performed depending on the conditions of the image.

FIG. 9 is a diagram illustrating a histogram of luminance before and after the histogram enhancement process when there is an object appearing dark in the image.

As illustrated in FIG. 9, when dust or air bubbles are included in the image, incident light is blocked, and thus a region having a low luminance value appears in the image, and a histogram D has not only a peak D_P1 of the background but also a peak D_P2 of the foreign matter. When such a histogram image is simply histogram-extended as described above, a shape of the histogram hardly changes before and after the correction process, as can be seen from the histogram D before the correction process and a histogram D1 after the correction process. That is, this is equivalent to the fact that the histogram enhancement process (correction process) is not performed, and there is a possibility that an image process such as discrimination between the background and bacteria in the image and feature value extraction cannot be performed with sufficient accuracy.

FIG. 10 is a diagram illustrating a histogram of luminance before and after the histogram enhancement process when almost no bacteria appear in the image.

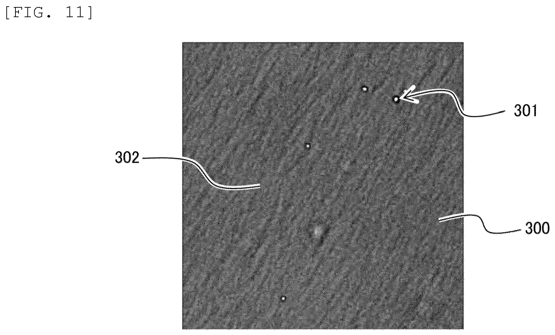

As shown in FIG. 10, a histogram E before correction has a steep peak near a center of a luminance range, but a peak of a histogram E1 after correction is gentle. FIG. 11 is a diagram illustrating an example of the image after the contrast enhancement process (correction process) as illustrated in FIG. 10. In FIG. 11, not only a contrast of the contour of the region of the bacteria 301 is enhanced, but also a region of a scratch 302 in the region of the background 300 is enhanced. That is, this is an excessive histogram enhancement process in which scratches or the like other than the bacteria 301 that is the inspection target are clearly shown in the image, and it can be said that this is not a valid correction process.

In the embodiment, in view of the above situation, a detection device and a detection method capable of correctly performing a correction process, even in a case where a particle density in an image greatly changes due to proliferation of bacteria and a foreign matter is contained, will be described below.

### (Bacterium Detection Process)

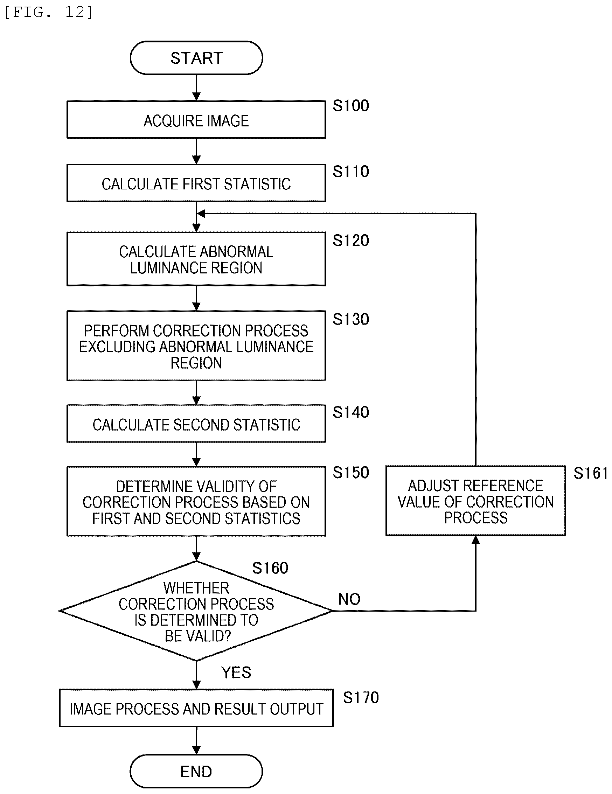

FIG. 12 is a flowchart illustrating a content of a bacterium detection process in the image-processing unit of the bacterium inspection device according to the embodiment.

As illustrated in FIG. 12, in the bacterium detection process, the image acquisition unit 161 of the image-processing unit 160 first acquires the image captured by the imaging unit 150 (step S100). Specifically, the image acquisition unit 161 receives the image acquired by the imaging unit 150 and stores the image in an internal memory (not illustrated) of the image-processing unit 160 or a memory (not illustrated) of the control device 200. In an image captured under preferable conditions, the inside of the region of bacteria often appears white and the contour often appears black, but the invention is not necessarily limited thereto. In the following description, it is assumed that the image is an 8-bit grayscale image, with a pixel value of 0 being black and a pixel value of 255 being white. The process according to the embodiment is also effective for a grayscale image other than 8-bit or an image with black and white reversed. It is also possible to apply the same process according to the embodiment to a color image by converting the color image into color spaces such as R, G, and B, or H, S, and V.

When the process of step S100 is completed, the statistic calculation unit 162 subsequently calculates a statistic (first statistic) related to the spread of the luminance value of the image acquired by the image acquisition unit 161 (step S110). Here, the statistic preferably indicates information on a pixel value histogram, that is, for example, a mean value and a standard deviation of luminance of all pixels. Information on the peak position of the histogram can be acquired from the mean value of all the pixels, and information on the spread of the peak can be acquired from the standard deviation of the histogram. The statistic is not necessarily to be as described above, and for example, a mode value may be used instead of the mean value of the luminance of all the pixels, and a half-value width of the peak may be used instead of the standard deviation.

When the process of step S120 is completed, the abnormal luminance region calculation unit 163 subsequently calculates a region having an abnormal luminance value of the image based on a result of comparison of the luminance value of the image with the predetermined luminance threshold, and excludes the calculated region from subsequent process targets (step S120). In other words, an abnormal luminance region derived from the foreign matter included in the image is calculated. Specifically, since the foreign matter often absorbs, reflects, or scatters illumination light, the luminance is often near a lower limit or an upper limit. Therefore, a lower limit threshold or an upper limit threshold of the luminance is set as the luminance threshold, and a pixel whose luminance value is less than the lower limit threshold or a pixel whose luminance value exceeds the upper limit threshold is extracted as a pixel in the abnormal luminance region. That is, the pixel to be extracted is excluded in the subsequent process. In the extraction of the pixel in the abnormal luminance region, both the lower limit threshold and the upper limit threshold of the luminance may be set, and the pixel having a luminance outside the respective thresholds may be excluded, or only one of the lower limit threshold and the upper limit threshold of the luminance may be set, and the pixel having a luminance outside the threshold may be excluded. In addition, the upper limit threshold and the lower limit threshold may be appropriately changed according to a state of a sample.

When the process of step S120 is completed, the correction process unit 164 subsequently performs the correction process of sharpening the image excluding the region having the abnormal luminance value (step S130). Specifically, the correction process is performed excluding the abnormal luminance region extracted in step S120. As an example of the correction process, for example, the contrast enhancement process by extension of the histogram is performed. At this time, when the lower limit value and the upper limit value of the saturation luminance are calculated by cumulative calculation, the abnormal luminance region is excluded from the cumulative calculation, and the influence of the foreign matter on the contrast enhancement is excluded.

When the process of step S130 is completed, the statistic calculation unit 162 subsequently calculates a statistic (second statistic) related to the spread of the luminance value of the image subjected to the correction process by the correction process unit 164 (step S140). That is, the second statistic of the pixel value is calculated for the image on which the correction process in step S130 has been performed. At this time, similarly to the first statistic, it is preferable to calculate the mean value and the standard deviation of the luminance of all the pixels.

When the process of step S140 is completed, the validity determination unit 166 subsequently determines the validity of the correction process by the correction process unit 164 based on the result of comparison of the ratio of the statistic (first statistic) of the image before the correction process and the statistic (second statistic) of the image after the correction process with the predetermined statistical ratio threshold (step S150). Specifically, first, a ratio between the pixel mean values before and after the correction process and a ratio between the standard deviations are calculated. A case where the ratio between the standard deviations is a high value indicates a case where the contrast enhancement process is excessive. For example, the ratio between the standard deviations is compared with a certain threshold (statistical ratio threshold: for example, 5), and when the ratio is equal to or larger than the threshold, it is determined that the contrast enhancement process is excessive, that is, the contrast enhancement process is not valid. The statistical ratio threshold is determined by a prior experiment or the like (described later). When the ratio between the mean values and the ratio between the standard deviations are close to 1, a case where the correction process is hardly effective is illustrated, and it is determined that the contrast enhancement process is excessively small, that is, the contrast enhancement process is not valid. Otherwise, it is determined that the contrast enhancement process is valid.

When the process of step S150 is completed, the validity determination unit 166 subsequently checks whether the correction process of the correction process unit 164 is determined to be valid (step S160).

When the result of determination in step S160 is NO, that is, when the validity determination unit 166 determines that the correction process of the correction process unit 164 is not valid, the adjustment unit 165 adjusts the reference value used for the correction process in the correction process unit 164 (step S161), and the process returns to step S120. That is, the process of step S160 is a process of performing an additional correction (step S130) and redetermination (step S161) as necessary. Specifically, when the contrast enhancement process is not valid (not appropriate), for example, when the contrast enhancement process is excessive, a reference value at the time of cumulative determination is set to a low value, and the intensity of the enhancement process is weakened, or the process proceeds to the next process (step S170) without performing the contrast enhancement process. When the contrast enhancement process is excessively small, the reference value at the time of the cumulative determination may be set to a high value, and the intensity of the enhancement process may be increased, or an abnormality flag may be added to the image by assuming that a foreign matter that cannot be removed in the process of step S120 appears in the image. In the embodiment, the process of steps S120 to S160 and S161 is repeatedly executed until it is determined that the correction process is valid, and the process is executed until the appropriate correction process is performed while sequentially changing the range of the abnormal luminance region and the strength of the contrast enhancement.

When the result of determination in step S160 is YES, that is, when the validity determination unit 166 determines that the correction process of the correction process unit 164 is valid, the image process output unit 167 determines the degree of proliferation of the fine particles based on the feature value of the image for which the correction process is determined to be valid, and outputs the result of determination to the control device 200 (step S170). Specifically, the image process output unit 167 executes a series of image process such as an edge extraction process, a process of comparing the luminance value with the neighboring pixel and detecting a maximum or minimum region, and binarization on the image for which the correction process has been completed, and calculates and outputs numerical information from the image. In a case of bacterium inspection, it is important to calculate the degree of proliferation of bacteria, and the number of regions recognized as bacteria in the image, an area or total area of the regions, and a feature value (for example, circularity, roundness, perimeter, convexity, aspect ratio) indicating shape information for each region are calculated.

Here, a method for setting the statistical ratio threshold used in the validity determination unit 166 in the process of step S150 of FIG. 12 will be described.

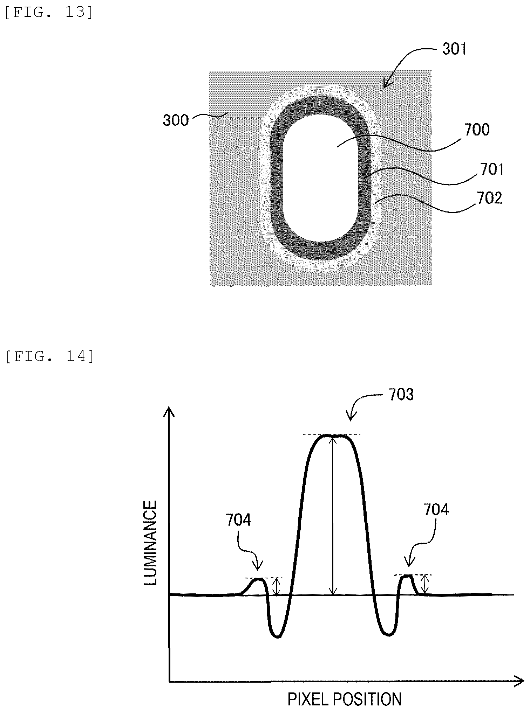

FIG. 13 is a diagram schematically illustrating a single bacterium in the image. FIG. 14 is a diagram illustrating a relationship between a position of a pixel in FIG. 13 and a luminance value.

In FIG. 13, compared to the region of the background 300, an inside 700 of the region of the bacteria 301 has a high luminance value, and a contour 701 of the region of the bacteria 301 has a low luminance value. A boundary 702 between the contour of the region of the bacteria 301 and the background has a luminance value slightly higher than that of the background. In such a case, a distribution of luminance values on a line crossing the region of the bacteria 301 in FIG. 13 is as illustrated in FIG. 14. As illustrated in FIG. 14, the distribution of the luminance values has a bilaterally symmetrical shape, and a minimum peak of the contour of the bacteria and a maximum peak 704 of the boundary between the bacteria and the background exist around the maximum peak 703 of the inside of the region of the bacteria 301, and an amplitude thereof gradually decreases. In the embodiment, the region of the bacteria is determined in the process of step S170, but for example, the maximum peak 703 of the inside of the region of the bacteria may be searched. On the other hand, when it is determined in the process of step S160 that the contrast enhancement process has been excessively performed, it is considered that the maximum peak 704 of the boundary between the bacteria and the background has also been amplified, and thus there is a possibility that the two maximum peaks cannot be discriminated by simple threshold determination. Therefore, the maximum peak 704 of the boundary between the bacteria and the background can be regarded as noise, and it is preferable to perform contrast enhancement within a range in which the noise does not affect the detection.

In the embodiment, a value of the noise is about 10. Since the noise is also increased when the contrast enhancement process is performed, for example, when the ratio between the standard deviations calculated in the process of step S140 is 5, the noise is (initial value 10) × (ratio of 5 between standard deviations) = 50. That is, in order to determine the region of the bacteria in the process of step S170, for example, when the threshold = 50 is adopted, the ring-shaped region of the boundary 702, which is noise, between the contour of the bacteria and the background is erroneously recognized as the bacteria. From the above, it can be said that it is preferable to adopt (threshold for determining bacterium region in step S170) ÷ (value of noise) as the threshold used for determining whether the contrast enhancement process is excessive. In the embodiment, the maximum peak 704 of the boundary between the region of the bacteria 301 and the background is dominant noise, but for example, when background noise of the region of the background 300 is large, an optimum threshold may be determined using a value of the background noise.

### (Effects)

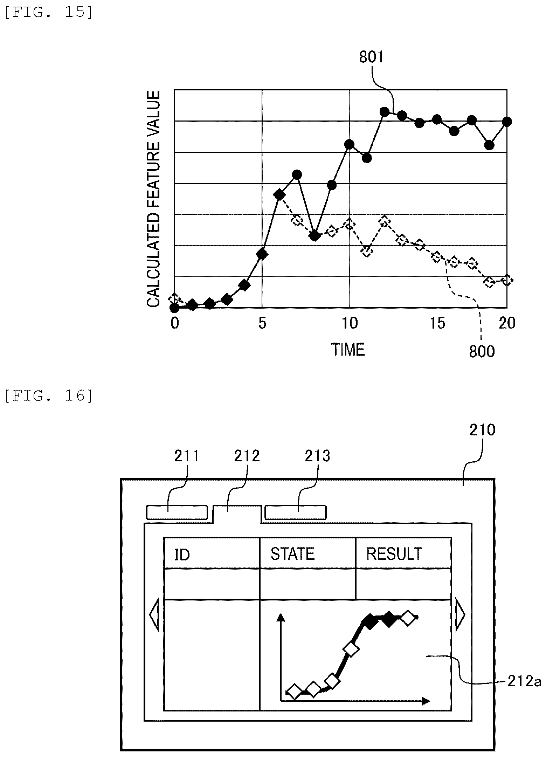

FIG. 15 is a diagram illustrating an effect of the image process in the image-processing unit.

In FIG. 15, a correction process and a binarization process are performed by the image-processing unit 160 on a clear bacterial image, which is acquired by the imaging system of the bacterium inspection device 100 according to the embodiment and proliferated normally, and the detected number of bacteria is organized into time-series.

In FIG. 15, data 800 is a result when the correction process in the related art is used, and data 801 is a result when the correction process determined to be appropriate by the embodiment is used.

As illustrated in FIG. 15, at a time 0, an output value of the data 800 is larger and indicates a high value of 10 times or more. This is because the contrast enhancement process is excessive when the number of bacteria is small, and the scratches on the well bottom are erroneously determined as bacteria.

After a time 7, the output value of the data 800 according to the related art is smaller and gradually decreases, whereas the data 801 according to the embodiment accurately indicates a state in which the bacteria increase and saturate with a change in the bacterial image obtained by acquiring expected normal proliferation of the bacteria. This is because, in the data 800 according to the related art, a foreign matter floating in the well appears in the image after the time 7, and the contrast enhancement process is excessively small, whereas, in the data 801 according to the embodiment, the contrast enhancement process can be performed at a valid intensity.

As described above, in the embodiment, by appropriately performing the contrast enhancement process, it is possible to perform an accurate image process even in a case where a concentration of a target object such as bacteria changes greatly and a foreign matter is included.

### (Result Output)

FIG. 16 is a diagram illustrating an example of an image process result output to the input and output unit of the control device.

FIG. 16 illustrates the result of the bacterium detection process of the image-processing unit 160, in other words, the result of the bacterium inspection process of the bacterium inspection device 100 (control device 200), and is displayed to a user by being displayed in a form of a graphical user interface (GUI) on, for example, the input and output unit 210 of the control device 200.

As illustrated in FIG. 16, the result of the bacterium inspection process includes, for example, a measurement condition display unit 211, a measurement result display unit 212, and a device state display unit 213 for switching a display content in a tab format.

The measurement result display unit 212 displays, as the result of the bacterium inspection process, for example, an ID of a specimen as an inspection target, a current measurement state of the specimen as the inspection target, a measurement result, and an output result 212a of the image process.

In a column of the measurement state of the specimen, it is preferable to display a measurement time up to the present, the time remaining until the measurement is completed, and an instruction to the user to take out the plate.

In a column of the measurement result, it is preferable to display the presence or absence of an error and a warning in the middle of measurement, and error information determined by the image process, for example, information such as the presence or absence of a foreign matter. It is also preferable to have a plot of a time-dependent change in numerical information calculated by the image process, which is useful for intuitively showing the degree of proliferation of bacteria to the user. Specifically, each rectangular point in this plot represents a point where measurement is executed, and if there is a foreign matter, a state may be clearly indicated, such as by being shown in a different color or shape from the others, and it is preferable that details of an error and the corresponding image can be output to the user by selecting the corresponding plot.

In the measurement condition display unit 211, conditions for performing bacterium inspection are displayed, and input and output are possible. For example, it is possible to display the measurement conditions of the inspection related to bacterial species serving as an inspection target, drugs, an inspection time, an imaging cycle of the images, or the like, and to receive outputs from the user.

In the device state display unit 213, information indicating a device state is displayed, and input and output are possible. For example, it is possible to display information about errors or warning of the device, information about temperature and humidity, opening and closing information about a device door, information about device maintenance, and various types of setting information about the device, and to receive outputs from the user.

As described above, in the embodiment, an inspection device includes: an imaging unit configured to capture an image of an inside of a container that retains a liquid containing fine particles; and an image-processing unit configured to perform an image process on the image captured by the imaging unit. The image-processing unit calculates a first statistic related to a spread of a luminance value of the image, excludes a region having an abnormal luminance value in the image based on a result of comparison of the luminance value of the image with a predetermined luminance threshold, performs a correction process of sharpening the image from which the region having the abnormal luminance value is excluded, calculates a second statistic related to the spread of the luminance value of the image subjected to the correction process, determines validity of the correction process based on a result of comparison of a ratio of the first statistic to the second statistic with a predetermined statistical ratio threshold, determines a degree of proliferation of the fine particles based on a feature value of the image for which the correction process is determined to be valid, and outputs a result of determination of the degree of proliferation. Therefore, it is possible to more accurately extract bacteria and detect a region in which bacteria corresponding to the degree of proliferation are present regardless of the difference in the degree of proliferation or the presence or absence of foreign matters other than the bacteria included in an image including a particulate object to be measured such as proliferating bacteria.

### <Second Embodiment>

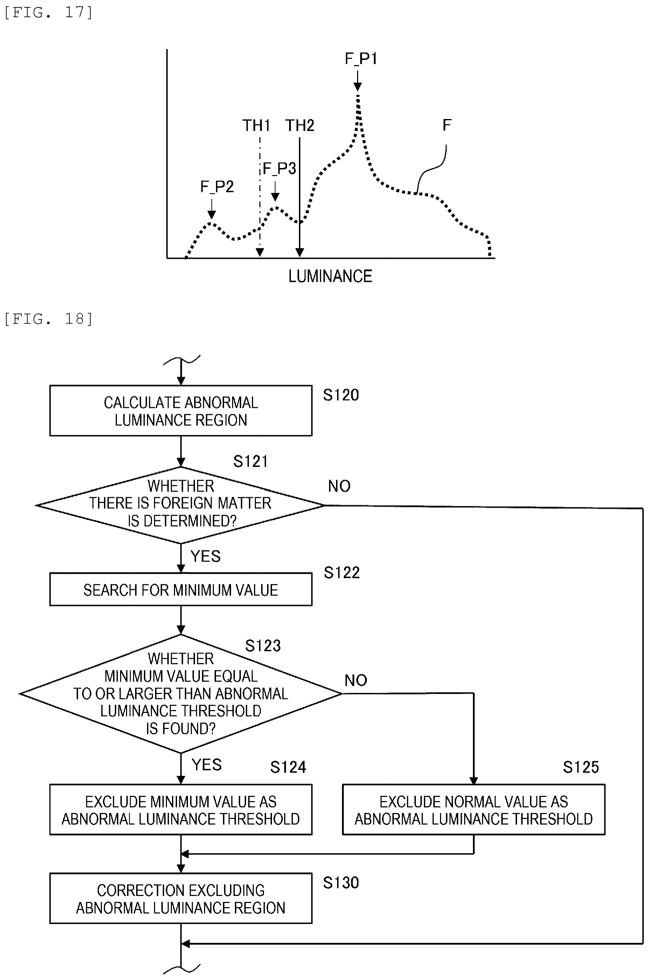

A second embodiment of the invention will be described with reference to FIGS. 17 and 18. In the drawings, the same members as those in the first embodiment are denoted by the same reference numerals, and description thereof will be omitted as appropriate.

In the embodiment, the threshold is changed according to the presence or absence of a foreign matter in an image, so that it is possible to cope with a case where more foreign matters appear in the image.

In the first embodiment, when the contrast enhancement process is performed, the number of pixels are accumulated in descending order of the luminance value, and a point where the accumulated value exceeds a reference is set as the lower limit value B1_min of the saturation luminance, and the number of pixels is accumulated in ascending order of the luminance value, and a point where the accumulated value exceeds the reference is set as the upper limit value B1_Max of the saturation luminance (see FIG. 8). This method is simple because it is only necessary to determine a certain reference value, and it is possible to exclude the foreign matter by combining with the calculation of the abnormal luminance region, but it is conceivable that an appropriate correction process cannot be performed in a state where more foreign matters appear in the image.

FIG. 17 is a diagram illustrating an example of a histogram of a luminance when many foreign matters appear in the image.

As illustrated in FIG. 17, in a histogram F, a peak F_P2 of a foreign matter and a peak F_P3 of a second foreign matter are present on the left side of a peak F_P1 of a background region. This means that there are regions corresponding to at least two foreign matters having different brightness in the image. The peak F_P2 of the foreign matter and the peak F_P3 of the second foreign matter cannot be clearly separated on the histogram F. In this case, when an intermediate luminance value between the peak F_P2 of the foreign matter and the peak F_P3 of the second foreign matter is selected as a threshold TH1 of an abnormal luminance, an influence of a pixel of the second foreign matter cannot be excluded. Therefore, in the embodiment, a method capable of more correctly excluding the foreign matter region and performing the contrast enhancement process will be described.

FIG. 18 is a flowchart illustrating a part of a content of an image process according to the embodiment.

FIG. 18 illustrates a process performed between the process of step S120 and the process of step S130 in the flowchart illustrated in FIG. 12 of the first embodiment. The following process can be performed for each of a lower limit and an upper limit of the saturation luminance, and a case where the process is performed for the lower limit of the saturation luminance will be described as an example.

When the process of step S120 (see FIG. 12) is completed, it is subsequently determined whether a foreign matter is included in the image based on the calculation result of the abnormal luminance region (step S121). For example, when it is assumed that an initial value of an abnormal luminance threshold is 40, a reference value is 1%, and an image size is 100 × 100 pixels, it is determined that there is a foreign matter in a case where there are 100 or more pixels having a luminance less than 40, and it is determined that there is no foreign matter in other cases.

When the result of determination in step S121 is NO, that is, when it is determined that there is no foreign matter, the process proceeds to step S140 (see FIG. 12).

When the result of determination in step S121 is YES, that is, when it is determined that there is a foreign matter, an appropriate abnormal luminance threshold is searched for (step S122). Specifically, a mode value of the histogram of the luminance value is first calculated, and the search is performed in descending order from the mode value to find a minimum value on the histogram. At this time, since a distribution of the histogram is not necessarily smooth, it is preferable to search for a minimum luminance value by calculating a first derivative and a second derivative of the histogram after executing a smoothing process or a low-pass filter process. In addition, a signal process such as curve fitting may be performed.

When the process of step S122 is completed, it is subsequently determined whether a minimum value equal to or larger than the abnormal luminance threshold is found (step S123).

When the result of determination in step S123 is YES, that is, when the minimum value is found and the minimum value is equal to or larger than the initial value of the abnormal luminance threshold, the found minimum value is replaced with the abnormal luminance threshold (step S124), and the process proceeds to step S130 (see FIG. 12).

When the result of determination in step S122 is NO, that is, when the minimum value is not found, or when the minimum value is less than the initial value of the abnormal luminance threshold even if the minimum value is found, or when the minimum value is another incorrect value, the abnormal luminance threshold is maintained at the initial value (step S125), and the process proceeds to step S130 (see FIG. 12).

Other configurations are the same as those of the first embodiment.

In the embodiment having the configuration described above, the same effect as that in the first embodiment can also be obtained.

Since it is possible to set an appropriate abnormal luminance threshold for each image and execute a correction process excluding an abnormal region, it is possible to select an appropriate threshold TH2 of an abnormal luminance even when there are a plurality of peaks of a foreign matter as in the histogram illustrated in FIG. 17, for example.

### <Appendix>

Functions of each embodiment can also be implemented by a software program code. In this case, a storage medium that records the program code is provided in a system or a device, and a computer (or CPU or MPU) of the system or the device reads the program code stored in the storage medium. In this case, the program code read from the storage medium implements the functions of the above-described embodiment, and the program code and the storage medium that stores the program code constitute the present disclosure. Examples of the storage medium for supplying such program codes include a flexible disk, a CD-ROM, a DVD-ROM, a hard disk, an optical disk, a magneto-optical disk, a CD-R, a magnetic tape, a nonvolatile memory card, and a ROM.

An operating system (OS) or the like operating on a computer may perform a part or all of actual processes based on an instruction of the program codes, and the functions of the embodiments described above may be implemented by the processes. Further, after the program codes read from the storage medium are written in a memory on the computer, a CPU or the like of the computer may perform a part or all of actual processes based on an instruction of the program codes, and the functions of the embodiments described above may be implemented by the processes.

Further, the software program code for implementing the functions of the embodiments may be stored, by distributing via a network, in a storage unit such as a hard disk or a memory of the system or the device or a storage medium such as a CD-RW or a CD-R, and the computer (or CPU or MPU) of the system or the device may read and execute the program code stored in the storage unit or the storage medium at the time of use.

It is necessary to understand that processes and techniques described here are not essentially related to any specific device, and can be implemented by any appropriate combination of components. Further, various types of devices for general purposes can be used according to the gist of the invention. It may be advantageous to construct a dedicated device to execute steps of the method described here. Various inventions can be formed by appropriately combining a plurality of components disclosed in the embodiment. For example, several components may be deleted from all components disclosed in the embodiment. Further, components belonging to different embodiments may be appropriately combined. Although the present disclosure has been described in relation to specific examples, the specific examples are used for description only, and are not intended to limit the present disclosure in all viewpoints. It is understood that there are many combinations of hardware, software, and firmware suitable for implementing the present disclosure for those skilled in the present field. For example, the above-described software can be implemented in a wide range of programs or script languages such as assembler, C/C++, perl, Shell, PHP, and Java (registered trademark).

Further, control lines and information lines considered to be necessary for description are shown in the embodiments described above, and not all control lines and information lines in a product are necessarily shown. All configurations may be connected to one another.

The present disclosure is not limited to the embodiments described above and examples, and includes various modifications. The embodiments described above are described in detail to describe the present disclosure in an easy-to-understand manner, and are not necessarily limited to including all the described configurations. A part of a configuration of a certain embodiment can be replaced with a configuration of another embodiment, and a configuration of another embodiment can also be added to a configuration of a certain embodiment. In addition, another configuration can be added to, deleted from, or replaced with a part of a configuration of each embodiment.

### Reference Signs List

100: bacterium inspection device
110: illumination unit
120: inspection plate
130: stage
140: objective lens
150: imaging unit
160: image-processing unit
161: image acquisition unit
162: statistic calculation unit
163: abnormal luminance region calculation unit
164: correction process unit
165: adjustment unit
166: validity determination unit
167: image process output unit
180: sample solution
190: humidification temperature adjustment unit
200: control device
210: input and output unit
211: measurement condition display unit
212: measurement result display unit
212a: output result
213: device state display unit
300: background
301: bacteria
302: scratch

## Claims

1. An inspection device comprising:
an imaging unit configured to capture an image of an inside of a container that retains a liquid containing fine particles; and
an image-processing unit configured to perform an image process on the image captured by the imaging unit, wherein
the image-processing unit
calculates a first statistic related to a spread of a luminance value of the image,
excludes a region having an abnormal luminance value in the image based on a result of comparison of the luminance value of the image with a predetermined luminance threshold,
performs a correction process of sharpening the image from which the region having the abnormal luminance value is excluded,
calculates a second statistic related to the spread of the luminance value of the image subjected to the correction process,
determines validity of the correction process based on a result of comparison of a ratio of the first statistic to the second statistic with a predetermined statistical ratio threshold,
determines a degree of proliferation of the fine particles based on a feature value of the image for which the correction process is determined to be valid, and
outputs a result of determination of the degree of proliferation.

2. The inspection device according to claim 1, wherein
the image-processing unit calculates, as the first statistic and the second statistic, a standard deviation of the luminance value of the image.

3. The inspection device according to claim 1, wherein
the image-processing unit calculates, as the first statistic and the second statistic, a half-value width of a histogram of the luminance value of the image.

4. The inspection device according to claim 1, wherein
the image-processing unit calculates, as the first statistic and the second statistic, a mean value of the luminance value of the image.

5. The inspection device according to claim 1, wherein
the correction process is a process of enhancing a contrast of shading of the image by extending a histogram of the luminance value of the image in a luminance value direction.

6. The inspection device according to claim 1, wherein
the correction process is a process of performing extreme value search in descending order or ascending order from a peak value of a histogram of the luminance value of the image, determining at least one or more luminance values at which the luminance value is a minimum value on the histogram, and extending the histogram of the luminance value of the image in a luminance value direction using the luminance value as a lower limit value or an upper limit value, thereby enhancing a contrast of shading of the image.

7. The inspection device according to claim 1, wherein
the image-processing unit cancels the correction process of the image for which the correction process is determined to be valid, or performs an additional correction process.

8. The inspection device according to claim 7, wherein
the correction process is a process of performing extreme value search in descending order or ascending order from a peak value of a histogram of the luminance value of the image, determining at least one or more luminance values at which the luminance value is a minimum value on the histogram, and extending the histogram of the luminance value of the image in a luminance value direction using the luminance value as a lower limit value or an upper limit value, thereby enhancing a contrast of shading of the image, and
as the additional correction process, a process of changing at least one of the lower limit value and the upper limit value when extending the histogram and enhancing the contrast of the shading of the image is performed.

9. The inspection device according to claim 1, wherein
the image-processing unit
executes a binarization process on the image for which the correction process is determined to be valid,
detects a region of the fine particles in the image by the binarization process,
calculates a feature value related to the number and shapes of the fine particles, and
determines the degree of proliferation of the fine particles based on the feature value.

10. An inspection method comprising:
a step of calculating a first statistic related to a spread of a luminance value of a captured image of an inside of a container that retains a liquid containing fine particles;
a step of excluding a region having an abnormal luminance value in the image based on a result of comparison of the luminance value of the image with a predetermined luminance threshold;
a step of performing a correction process of sharpening the image from which the region having the abnormal luminance value is excluded;
a step of calculating a second statistic related to the spread of the luminance value of the image subjected to the correction process;
a step of determining validity of the correction process based on a result of comparison of a ratio of the first statistic to the second statistic with a predetermined statistical ratio threshold;
a step of determining a degree of proliferation of the fine particles based on a feature value of the image for which the correction process is determined to be valid; and
a step of outputting a result of determination of the degree of proliferation.
